# EUROPEAN PATENT APPLICATION

(11) **EP 4 722 697 A1**
(43) Date of publication of application: **08.04.2026**
(21) Application number: 24815811.5
(22) Date of filing: 27.05.2024
(51) Int. Cl.: G01N 21/65, G01N 21/552, G01J 3/44, G01N 33/574, B82Y 15/00, B82Y 40/00

(54) **STRUCTURE FOR SURFACE-ENHANCED RAMAN SCATTERING SPECTROSCOPY, MANUFACTURING METHOD THEREFOR, AND DIAGNOSIS METHOD USING SAME**

(30) Priority: 26.05.2023 KR 20230068519; 30.10.2023 KR 20230146290; 01.12.2023 KR 20230172651
(71) Applicant: Emocog Inc., Seoul 08708 (KR)
(72) Inventor: HWANG, Jaeho, Suwon-si, Gyeonggi-do 16512 (KR); KIM, Jieun, Seoul 07289 (KR)
(74) Representative: Zacco Sweden AB
(86) International application number: PCT/KR2024/007124
(87) International publication number: WO 2024/248435

(57) **Abstract**

The present disclosure relates to a structure for surface-enhanced Raman scattering spectroscopy, a method of preparing the same, and a diagnostic method using the same. According to the structure for surface-enhanced Raman scattering spectroscopy, the method of preparing the same, and the diagnostic method using the same, of the present disclosure, a structure for surface-enhanced Raman scattering spectroscopy and a method of preparing the same may be provided, which can be used even when biomarkers with excellent diagnostic performance among blood indicators do not exist, as in the case of intractable cancer, such as pancreatic cancer, and intractable diseases. Also, a diagnostic method and diagnostic system using surface-enhanced Raman scattering spectroscopy may be provided by using the structure for surface-enhanced Raman scattering spectroscopy.

## Description

### Technical Field

The present disclosure relates to a structure for surface-enhanced Raman scattering spectroscopy, a method of preparing the same, and a diagnostic method using the same.

### Background Art

Surface-enhanced Raman scattering (SERS) spectroscopy is designed to complement Raman scattering spectroscopy, which has weak signals and low reproducibility, and is spectroscopy using the phenomenon in which the Raman scattering intensity of molecules adsorbed on the surface of metal nanostructures, such as gold and silver, increases dramatically by 10⁶ to 10⁸ times or more.

SERS spectroscopy enables the acquisition of large amounts of information with a single measurement, is an ultra-sensitive technique capable of directly measuring a single molecule, and enables direct measurement of information about molecular vibration states or molecular structures, and thus is recognized as a powerful analytical method for chemical/biological/biochemical analysis.

Recently, SERS spectroscopy has been attracting attention as a method of precisely obtaining comprehensive information about biomolecules from biological analytical samples. However, previously proposed detection methods for biomolecules, using SERS spectroscopy require specific binding, which presents challenges for the screening and early diagnosis of intractable cancers, such as pancreatic cancer, and intractable diseases, for which biomarkers with excellent diagnostic performance among blood indicators do not exist.

Therefore, there is a need for a diagnostic method and diagnostic system using SERS spectroscopy that can be used even when biomarkers with excellent diagnostic performance among biological indicators do not exist, as in the case of intractable cancers, such as pancreatic cancer, and intractable diseases.

Meanwhile, the matters described as background art should only be understood as enhancing the understanding of the background of the present disclosure, and should not be taken as an acknowledgement that these matters correspond to prior art already known to those of ordinary skill in the art.

### Disclosure of Invention

### Technical Problem

The technical problem to be achieved by the present disclosure is to provide a structure for surface-enhanced Raman scattering spectroscopy and a method of preparing the same, which can be used even when biomarkers with excellent diagnostic performance among blood indicators do not exist, as in the case of intractable cancers, such as pancreatic cancer, and intractable diseases.

The technical problem to be achieved by the present disclosure is to provide a disease diagnostic method and diagnostic system using surface-enhanced Raman scattering spectroscopy, which can be used even when biomarkers with excellent diagnostic performance among biological indicators do not exist.

Also, the technical problem to be achieved by the present disclosure is to provide a diagnostic method and diagnostic system using surface-enhanced Raman scattering spectroscopy, which have high repeatability and reproducibility and high convenience of distribution and storage.

The technical problems to be achieved by the present disclosure are not limited to the technical problems mentioned above, and other unmentioned technical problems will become apparent from the following description to those of ordinary skill in the art to which the present disclosure pertains.

### Solution to Problem

To achieve the technical problem, an embodiment of the present disclosure provides a structure for surface-enhanced Raman scattering spectroscopy, including a silica shell layer having an accommodation space therein and a nanoparticle including a plasmonic metal, wherein the nanoparticle is arranged in a region of the accommodation space in the silica shell layer.

To achieve the technical problem, another embodiment of the present disclosure provides a method of preparing a structure for surface-enhanced Raman scattering spectroscopy, including preparing a solution in which nanoparticles are dispersed and forming a silica shell layer that surrounds each of the nanoparticle dispersed in the solution.

To achieve the technical problem, an embodiment of the present disclosure provides a disease diagnostic method using surface-enhanced Raman scattering spectroscopy, including acquiring a first surface-enhanced Raman scattering spectroscopic signal from a biological fluid extracted from a patient, irradiating, with light, a diagnostic solution in which metal nanostructures, each of which includes a biomolecular material in a region of the inside thereof, are dispersed, to acquire a second surface-enhanced Raman scattering spectroscopic signal from the regions of the metal nanostructures, and identifying a disease of the patient by comparing the first surface-enhanced Raman scattering spectroscopic signal and the second surface-enhanced Raman scattering spectroscopic signal.

To solve the technical problem, another embodiment of the present disclosure provides a disease diagnostic system using surface-enhanced Raman scattering spectroscopy, including a container unit in which a diagnostic solution is placed, an irradiation section that irradiates the diagnostic solution placed in the container unit with light and generates a surface-enhanced Raman scattering spectroscopic signal in a region of a metal nanostructure included in the diagnostic solution, and a diagnostic unit that analyzes the generated surface-enhanced Raman scattering spectroscopic signal.

### Advantageous Effects of Invention

According to an embodiment of the present disclosure, a structure for surface-enhanced Raman scattering spectroscopy and a method of preparing the same may be provided, the structure being capable of providing a surface-enhanced Raman scattering spectroscopic signal in a manner non-specific to a bimolecular material.

Also, according to an embodiment of the present disclosure, a structure for surface-enhanced Raman scattering spectroscopy and a method of preparing the same may be provided, which can be used even when biomarkers with excellent diagnostic performance among blood indicators do not exist, as in the case of intractable cancers, such as pancreatic cancer, and intractable diseases.

According to an embodiment of the present disclosure, a diagnostic method and diagnostic system using surface-enhanced Raman scattering spectroscopy may be provided, which can be used even when biomarkers with excellent diagnostic performance among biological indicators do not exist.

Also, according to an embodiment of the present disclosure, a diagnostic method and diagnostic system using surface-enhanced Raman scattering spectroscopy may be provided, which have high repeatability and reproducibility and high convenience of distribution and storage.

The effects of the present disclosure are not limited to the effects described above, and should be understood to include all effects that can be inferred from the detailed description of the present disclosure or the configurations of the disclosure set forth in the claims.

### Brief Description of Drawings

FIGS. 1A to 1E are conceptual views illustrating a structure for surface-enhanced Raman scattering spectroscopy, according to an embodiment of the present disclosure.
FIGS. 2A and 2B are conceptual views illustrating a diagnostic solution for surface-enhanced Raman scattering spectroscopy, according to an embodiment.
FIGS. 3A and 3B are flowcharts illustrating a method of preparing a structure for surface-enhanced Raman scattering spectroscopy, according to an embodiment.
FIGS. 4A and 4B are flowcharts illustrating a diagnostic method using surface-enhanced Raman scattering spectroscopy, according to an embodiment.
FIGS. 5A to 5C are block diagrams illustrating a diagnostic system according to an embodiment.
FIGS. 6A and 6B are TEM images of a structure for surface-enhanced Raman scattering spectroscopy, according to an embodiment.
FIG. 7 illustrates graphs showing Raman scattering experimental results in the presence or absence of a biomaterial in a structure, according to an embodiment.
FIG. 8 is a graph showing experimental results for the difference between Raman spectra with or without ethanol pretreatment, according to an embodiment.
FIG. 9 is a graph confirming a surface-enhanced Raman scattering spectroscopic signal of a diagnostic solution according to an embodiment.

### List of Reference Numerals for Major Elements

1: Diagnostic solution
10: Structure
11: Nanoparticle
12: Silica shell layer
20: Dispersion
100: Diagnostic system
110: Container unit
111: Core
112: Second plasmonic metal shell layer
113: Polymer coating layer
120: Irradiation section
130: Diagnostic unit
140: Data storage unit

### Best Mode for Carrying out the Invention

An embodiment of the present disclosure may provide a structure for surface-enhanced Raman scattering spectroscopy, including: a silica shell layer having an accommodation space therein; and a nanoparticle including a plasmonic metal, wherein the nanoparticle is arranged in a region of the accommodation space in the silica shell layer.

In an embodiment of the present disclosure, a structure for surface-enhanced Raman scattering spectroscopy may be provided,wherein the nanoparticle may include: a core including a first plasmonic metal; and a second plasmonic metal shell layer disposed to surround the core and including a second plasmonic metal, wherein the second plasmonic metal shell layer has a symmetrical structure with respect to a center of the second plasmonic metal shell layer.

In an embodiment of the present disclosure, the structure may be a structure for surface-enhanced Raman scattering spectroscopy, including a biomolecular material arranged in the accommodation space.

In an embodiment of the present disclosure, a structure for surface-enhanced Raman scattering spectroscopy may be a structure in which the first plasmonic metal and the second plasmonic metal each independently include any one or more selected from the group consisting of Au, Ag, Cu, Al, W, Pt, Ni, and Pd.

In an embodiment of the present disclosure, a structure for surface-enhanced Raman scattering spectroscopy may be a structure in which a width of a plasmon resonance peak on a surface of the second plasmonic metal isnarrower than a width of a plasmon resonance peak on a surface of the first plasmonic metal.

In an embodiment of the present disclosure, a structure for surface-enhanced Raman scattering spectroscopy may be a structure in which the second plasmonic metal shell layer may have a regular polyhedral structure.

Another embodiment of the present disclosure may provide a method of preparing a structure for surface-enhanced Raman scattering spectroscopy, including: preparing a solution in which nanoparticles are dispersed; and forming a silica shell layer that surrounds each of the nanoparticle dispersed in the solution.

In an embodiment of the present disclosure, a method for manufacturing a structure for surface-enhanced Raman scattering spectroscopy may be provided, wherein the preparation of the solution may include forming a mixed solution by mixing a first solution and a second solution, the first solution including a first precursor compound including a first plasmonic metal and the second solution including a second precursor compound including a second plasmonic metal, wherein the nanoparticle includes a core including a first plasmonic metal and a second plasmonic metal shell layer which is disposed to surround the core and includes a second plasmonic metal, and the second plasmonic metal shell layer has a symmetrical structure with respect to a center of the second plasmonic metal shell layer.

In an embodiment of the present disclosure, the method for manufacturing a structure for surface-enhanced Raman scattering spectroscopy may further include mixing the mixed solution with a coating solution including a water-soluble polymeric compound.

In an embodiment of the present disclosure, a method for manufacturing a structure for surface-enhanced Raman scattering spectroscopy may be provided, wherein the formation of the silica shell layer may include mixing the mixed solution with a third solution and a fourth solution, the third solution including a biomolecular material and the fourth solution including a silica precursor compound, wherein the silica shell layer is formed such that the nanoparticle and at least a part of the biomolecular material are arranged together inside the silica shell layer.

Another embodiment of the present disclosure may provide a disease diagnostic method using surface-enhanced Raman scattering spectroscopy, including: acquiring a first surface-enhanced Raman scattering spectroscopic signal from a biological fluid extracted from a patient; irradiating, with light, a diagnostic solution in which metal nanostructures, each of which includes a biomolecular material in a region of the inside thereof, are dispersed, to acquire a second surface-enhanced Raman scattering spectroscopic signal from the regions of the metal nanostructures; and identifying a disease of the patient by comparing the first surface-enhanced Raman scattering spectroscopic signal and the second surface-enhanced Raman scattering spectroscopic signal.

In an embodiment of the present disclosure, a disease diagnostic method using surface-enhanced Raman scattering spectroscopy may be a method in which the structure for surface-enhanced Raman scattering spectroscopy may further include: a silica shell layer having an accommodation space therein; and a nanoparticle including a plasmonic metal, wherein the nanoparticle and the biomolecular material are arranged in the accommodation space in the silica shell layer.

In an embodiment of the present disclosure, a disease diagnostic method using surface-enhanced Raman scattering spectroscopy may be a method in which the biomolecular material may include two or more types of biomolecular materials.

In an embodiment of the present disclosure, a disease diagnostic method using surface-enhanced Raman scattering spectroscopy may be a method in which the diagnostic method may further include organizing, into a database, the first surface-enhanced Raman scattering spectroscopic signal acquired from the biological fluid extracted from a patient.

In an embodiment of the present disclosure, a disease diagnostic method using surface-enhanced Raman scattering spectroscopy may be a method in which the diagnostic method may further include acquiring a surface-enhanced Raman scattering spectroscopic signal for the biomolecular material and organizing, into a database, information about the surface-enhanced Raman scattering spectroscopic signal for the biomolecular material.

Another embodiment of the present disclosure may provide a disease diagnostic system using surface-enhanced Raman scattering spectroscopy, including: a container unit in which a diagnostic solution is placed; an irradiation section that irradiates the diagnostic solution placed in the container unit with light and generates a surface-enhanced Raman scattering spectroscopic signal from a region of a metal nanostructure included in the diagnostic solution; and a diagnostic unit that analyzes the generated surface-enhanced Raman scattering spectroscopic signal.

In an embodiment of the present disclosure, a disease diagnostic system using surface-enhanced Raman scattering spectroscopy may be a system in which the structure for surface-enhanced Raman scattering spectroscopy may further include: a silica shell layer having an accommodation space therein; and a nanoparticle including a plasmonic metal, wherein the nanoparticle and the biomolecular material are arranged in the accommodation space in the silica shell layer.

In an embodiment of the present disclosure, a disease diagnostic system using surface-enhanced Raman scattering spectroscopy may be a system in which the biomolecular material may include two or more types of biomolecular materials.

In an embodiment of the present disclosure, a disease diagnostic system using surface-enhanced Raman scattering spectroscopy may be a system in which the diagnostic system may further include a data storage unit that acquires the surface-enhanced Raman scattering spectroscopic signal and stores spectroscopic information about the surface-enhanced Raman scattering spectroscopic signal.

### Mode for the Invention

Hereinafter, the present disclosure will be described with reference to the accompanying drawings. However, the present disclosure may be embodied in many different forms, and thus is not limited to the embodiments set forth herein. To clearly explain the present disclosure in the drawings, parts unrelated to the explanation are omitted, and like elements denote like reference numerals throughout the specification.

In the drawings, the sizes of elements may be exaggerated or reduced for convenience of explanation. For example, the size and thickness of each element are arbitrarily illustrated in the drawings for convenience of explanation, and the following embodiments are not necessarily limited thereto.

The terms first, second, A, B, and the like may be used to describe various elements, but the elements should not be limited by these terms. These terms are only used to distinguish one element from another element. For example, a first element may be named a second element without departing from the scope of the present disclosure, and similarly, the second element may also be named the first element. The term "and/or" includes any and all combinations of one or more of the associated listed items.

Throughout the specification, when a portion is referred to as being "connected to (linked to, in contact with, or coupled to)" another portion, it includes not only a case in which the portion is "directly connected" to the other portion, but also a case in which the portion is "indirectly connected" to the other portion with another element present therebetween.

In addition, when a portion is referred to as "including" a certain portion, it means a case where the portion may further include other elements, rather than precluding other elements, unless otherwise specifically stated herein.

The terms used herein are used to only describe specific embodiments and are not intended to limit the present disclosure. An expression in the singular includes an expression in the plural unless the content clearly indicates otherwise.

It should be understood that terms used herein, such as "include" and "have," are used to indicate the presence of stated features, numbers, steps, operations, elements, parts, or a combination thereof without excluding in advance the possibility of the presence or addition of one or more other features, numbers, steps, operations, elements, parts, or combinations thereof.

When a portion is referred to as being "on" or "on" another portion, it includes not only a case in which the portion is directly on the other portion, but also a case in which an intervening portion is present therebetween.

Previously proposed detection methods for biomolecules, using surface-enhanced Raman scattering spectroscopy include methods in which specific binding occurs between biomolecules and metal nanoparticles, and consequently, surface-enhanced Raman scattering spectroscopic signals are formed at the sites. Thus, even when surface-enhanced Raman scattering spectroscopy is used it was difficult to use surface-enhanced Raman scattering spectroscopy for diseases, such as pancreatic cancer, for which biomarkers having excellent diagnostic performance do not exist.

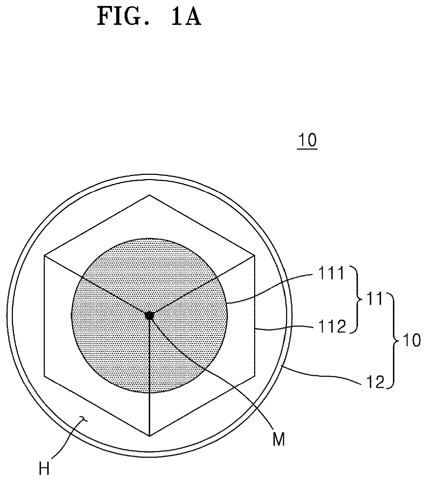

FIGS. 1A to 1E are conceptual views illustrating a structure for surface-enhanced Raman scattering spectroscopy, according to an embodiment of the present disclosure.

Referring to FIG. 1A, to solve the above-described technical problem, an embodiment of the present disclosure provides: a structure 10 for surface-enhanced Raman scattering spectroscopy (hereinafter, referred to as a "structure"), including a nanoparticle 11 and a silica shell layer 12; a composition for surface-enhanced Raman scattering spectroscopy in which the structures 10 are dispersed; and methods of preparing the same.

In an optional embodiment, the silica shell layer 12 may include therein biomolecular materials B, which will be described below, and through the structure, it is possible to acquire surface-enhanced Raman scattering spectroscopic signals for a considerable number of biomolecular materials B that are non-specifically arranged inside the structure 10, without excluding specific biomolecular materials B.

Furthermore, it is possible to provide a diagnostic kit having excellent diagnostic performance even for diseases for which biomarkers with excellent spectroscopic performance do not exist, by organizing, into a database, surface-enhanced Raman scattering spectroscopic signals by using the structure 10 and a composition for surface-enhanced Raman scattering spectroscopy in which the structures 10 are dispersed.

Hereinafter, the structure 10 will be described.

In an embodiment, the nanoparticle 11 may include a plasmonic metal. A surface-enhanced Raman scattering spectroscopic signal may be acquired by an increased Raman scattering signal through the plasmon phenomenon at a local surface in case that molecules or particles exist near a surface of a plasmonic metal.

The term "plasmonic metal" used herein may refer to a metal that is excited in a magnetic field, and examples thereof include Au, Ag, Cu, Al, W, Pt, Ni, Pd, and the like.

In an embodiment, the nanoparticle 11 may include a core 111 including a first plasmonic metal, and a second plasmonic metal shell layer 112 disposed to surround the core 111 and including a second plasmonic metal. As described above, the nanoparticle 11 has a core-shell structure, and thus the nanoparticle 11 having both the properties of the first plasmonic metal and the properties of the second plasmonic metal may be provided.

In an embodiment, the second plasmonic metal shell layer 112 may have a symmetrical structure with respect to a center M of the second plasmonic metal shell layer 112. As in the above-described structure, the second plasmonic metal shell layer 112 may be formed so as to surround the core 111 and have a symmetrical structure, and thus the surface area between the nanoparticle 11 and the biomolecular material B may be increased, thereby maximizing a surface-enhanced Raman scattering spectroscopic signal.

In an embodiment, the second plasmonic metal shell layer 112 may have a regular polyhedral structure, and a regular polyhedron may include, for example, at least one of a regular cube, a regular octahedron, a regular dodecahedron, and a regular icosahedron.

In an embodiment, the core 111 may include a first plasmonic metal, and as the first plasmonic metal, any metal capable of providing a surface-enhanced Raman scattering spectroscopic signal through a plasmon phenomenon on the surface should be constructed as falling within the scope of the present disclosure. For example, the first plasmonic metal may be, but is not limited to, Au, Ag, Cu, Al, W, Pt, Ni, and Pd.

In an embodiment, the core 111 may have an average particle diameter (D₅₀) of 50 nm or less. In another embodiment, the range may be 0 to 50, 5 to 50, 10 to 50, 15 to 50, 20 to 50, 25 to 50, 30 to 50, 35 to 50, 40 to 50, 45 to 50, 0 to 45, 5 to 45, 10 to 45, 15 to 45, 20 to 45, 25 to 45, 30 to 45, 35 to 45, 40 to 45, 0 to 40, 5 to 40, 10 to 40, 15 to 40, 20 to 40, 25 to 40, 30 to 40, 35 to 40, 0 to 35, 5 to 35, 10 to 35, 15 to 35, 20 to 35, 25 to 35, 30 to 35, 0 to 30, 5 to 30, 10 to 30, 15 to 30, 20 to 30, 25 to 30, 0 to 25, 5 to 25, 10 to 25, 15 to 25, or 20 to 25.

In case that the average particle diameter is within the above range and the structures 10 are arranged in a dispersion 20, which will be described below, an excellent Raman scattering effect may be provided.

In an embodiment, the core 111 may have a diameter in at least one direction of 10 nm to 50 nm. Due to having the above-described structure, in case that the structures 10 are arranged in the dispersion 20, which will be described below, an excellent Raman scattering effect may be provided.

In an embodiment, the second plasmonic metal shell layer 112 may include a second plasmonic metal, a width of a plasmon resonance peak on a surface of the second plasmonic metal may be narrower than a width of a plasmon resonance peak on a surface of the first plasmonic metal. As in the above-described structure, by making the width of a plasmon resonance peak on a surface of the second plasmonic metal narrower, in case that the structures 10 are dispersed in the dispersion 20, which will be described below, higher sensitivity may be provided in case that a Raman scattering signal is acquired.

In an optional embodiment, the width of a plasmon resonance peak on a surface of the first plasmonic metal and the width of a plasmon resonance peak on a surface of the second plasmonic metal may be measured under the same conditions.

In an embodiment, the second plasmonic metal shell layer 112 may include a second plasmonic metal. For example, the second plasmonic metal may be, but is not limited to, Au, Ag, Cu, Al, W, Pt, Ni, and Pd.

In an embodiment, the core 111 may include gold (Au), and the second plasmonic metal shell layer 112 may include silver (Ag). Ag has a light-gathering effect that is at least five times stronger than Au, and the structure 10 including the nanoparticle 11 having the above-described structure may provide an excellent surface-enhanced Raman scattering spectroscopic signal.

In an embodiment, the second plasmonic metal shell layer 112 may have a thickness in at least one direction of 10 nm to 70 nm. In another embodiment, the second plasmonic metal shell layer 112 may have a thickness of 30 nm to 70 nm, 40 nm to 70 nm, 50 nm to 70 nm, 60 nm to 70 nm, 30 nm to 60 nm, 40 nm to 60 nm, or 50 nm to 60 nm. In case that the thickness of the second plasmonic metal shell layer 112 exceeds 70 nm, the size of the nanoparticle 11 arranged inside the structure 10 becomes excessively large, which may decrease the reproducibility of surface-enhanced Raman scattering. In case that the thickness of the second plasmonic metal shell layer 112 is less than 30 nm, the gap between the second plasmonic metal shell layer 112 and the core 111 becomes too narrow, resulting in decreased reproducibility of surface-enhanced Raman scattering.

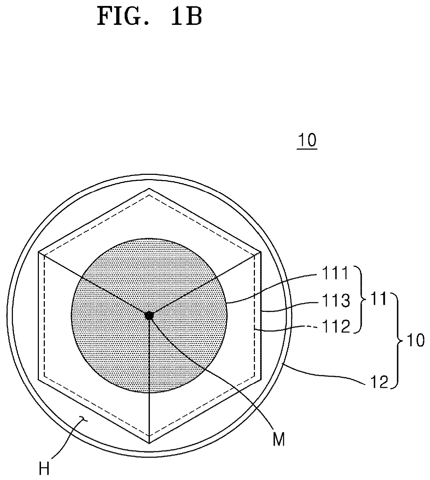

Referring to FIG. 1B, in an embodiment, the nanoparticle 11 may include a polymer coating layer 113 that is formed outside the second plasmonic metal shell layer 112 and surrounds the second plasmonic metal shell layer 112. The polymer coating layer 113 may allow the surfaces of the nanoparticles 11 to be negatively charged and prevent aggregation of the nanoparticles 11.

Due to having the above-described structure, in a step before the formation of the silica shell layer 12 in the method of preparing the structure 10, aggregation of the nanoparticles 11 may be prevented, and furthermore, the silica shell layer 12 may be formed more easily.

In an embodiment, cetyltrimethylammonium chloride (CTAC), which is a surfactant, and polyvinylpyrrolidone (PVP), which is a polymer, may be applied onto a surface of the metallic nanoparticle 11. CTAC can immobilize charged molecules to the surface of the nanoparticle 11 through electrostatic attraction, and PVP may play a role in immobilizing various biomolecules to the surface of the nanoparticle 11 through van der Waals attraction and chelation action. Thus, CTAC and PVP, which are applied onto the surface of the metallic nanoparticle 11, immobilize biomolecules in blood on the surface of the nanoparticle in the silica shell layer formation process, thereby allowing the biomolecules in blood to be easily positioned in the accommodation space in the silica shell layer.

In an embodiment, the polymer coating layer 113 is not particularly limited, and it should be interpreted that any polymer coating layer that can be easily selected as the polymer coating layer 113 by a person skilled in the art falls within the scope of the present disclosure.

In some embodiments, the polymer coating layer 113 may include a water-soluble polymeric compound. Examples of water-soluble polymeric compounds include, but are not limited to, polyvinylpyrrolidone, polyvinyl alcohol, polyfluorosulfonate, hydroxyethyl cellulose, hydroxypropyl cellulose, cellulose acetate, and polyamide.

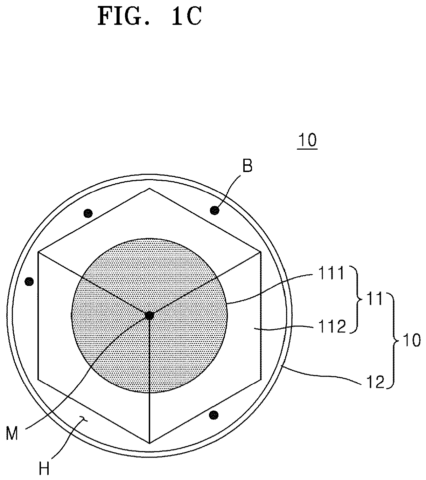

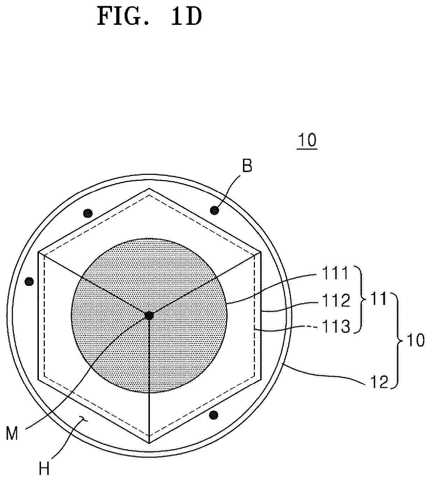

Referring to FIGS. 1C and 1D, in an embodiment, the structure 10 may further include a biomolecular material B arranged in an accommodation space H. As in the above-described structure, the nanoparticle 11 and the biomolecular material B may be arranged together inside the silica shell layer 12, and thus the biomolecular material B and the nanoparticle 11 that are arranged in the accommodation space H may come into contact with each other, and as described below, a surface-enhanced Raman scattering spectroscopic signal may be acquired from the surface of the nanoparticle 11.

The term "biomolecular material" as used herein refers to molecules and/or ions present in living organisms, and may refer to molecules and/or ions that are synthesized and/or produced during general biological processes such as cell division, morphogenesis, or development.

The biomolecular material B may include not only proteins, carbohydrates, lipids, nucleic acids, and minerals, but also low-molecular substances such as primary metabolites, secondary metabolites, and natural products, and may include both endogenous and exogenous substances. The biomolecular material B may be, for example, CA 19-9, amyloid beta40, and amyloid beta42 in plasma, but the present disclosure is not limited to these examples.

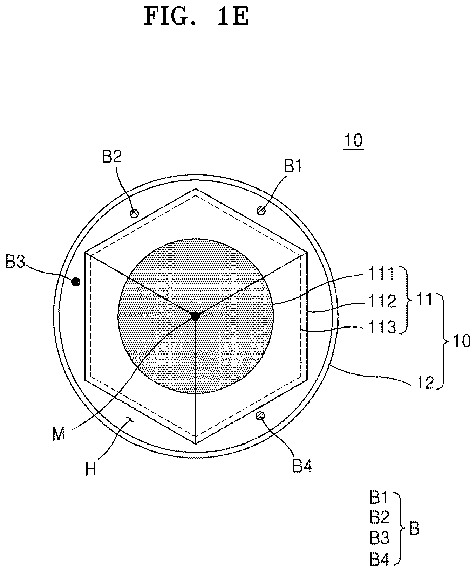

In some embodiments, as in the above-described structure, the structure 10 according to an embodiment may include the nanoparticle 11 that non-specifically causes a surface plasmon resonance phenomenon with the biomolecular materials B, thereby forming a peak. In an optional embodiment, referring to FIG. 1E, the structure 10 may include different biomolecular molecules B therein. Through the above structure, a plurality of surface-enhanced Raman scattering spectroscopic signals may be acquired from even a single structure 10.

In some embodiments, the plurality of acquired surface-enhanced Raman scattering spectroscopic signals may be organized into a database (DB) through a plurality of measurements, and specific peaks of diseases may be measured on the basis of the signals organized into a DB. Furthermore, when used in diagnostic kits and the like, the effect of accurately diagnosing diseases may be provided.

In an embodiment, the structure 10 may include two or more types of biomolecular materials B. By separating and extracting the upper or lower region of a biological fluid treated with a pretreatment solution, the dimensions of the overall acquired surface-enhanced Raman scattering spectroscopic signals may be diversified, and thus it is possible to organize a more detailed database for a plurality of different biomolecular materials B. Ultimately, it is possible to provide the structure 10 capable of more accurately acquiring surface-enhanced Raman scattering spectroscopic signals.

As described above, referring back to FIGS. 1A to 1E, in an embodiment, the structure 10 may include the silica shell layer 12. The silica shell layer 12 may have the accommodation space H therein, and the nanoparticle 11 and the biomolecular material B may be arranged in a region of the accommodation space H in the silica shell layer 12.

In case that nanoscale particles are dispersed in a liquid or the like, the particles may aggregate in the dispersion 20 through interactions (e.g., attractions) between the particles. However, in the case of including the silica shell layer 12 that surrounds each of the nanoparticles 11 as in the above-described structure, the silica shell layer 12 may prevent interactions between the nanoparticles 11, thus preventing aggregation of the particles, and furthermore, it is possible to provide the effect of stabilizing measurement results by increasing a surface-enhanced Raman scattering spectroscopic signal or increasing the reproducibility of the surface-enhanced Raman scattering spectroscopic signal.

The structure 10 according to an embodiment does not necessarily require a substrate, location, or probe that specifically binds to the biomolecule materials (B), and thus, in case that the biomolecular materials are located on or come into contact with the surface of the nanoparticle 11, surface-enhanced Raman scattering spectroscopic signals for a considerable number of the biomolecular materials B arranged in the structure 10 may be measured.

As described above, the measured surface-enhanced Raman scattering spectroscopic signals may be organized into a database, and ultimately, a diagnostic kit for diagnosing diseases even when biomarkers do not exist may be provided.

In an embodiment, the silica shell layer 12 may refer to a shell layer formed of silicon (Si) and oxygen (O) as main components, a material thereof is not particularly limited, and it should be interpreted that any silica shell layer that can be easily selected as the silica shell layer 12 by a person skilled in the art falls within the scope of the present disclosure. For example, the silica shell layer 12 may be the silica shell layer 12 formed by a polymerization reaction of TEOS, but the present disclosure is not limited to the example.

In an embodiment, the silica shell layer 12 may have a thickness of 20 to 30 nm, and in another optional embodiment, 20 to 29, 20 to 28, 20 to 27, 20 to 26, 20 to 25, 20 to 24, 20.5 to 30, 20.5 to 29, 20.5 to 28, 20.5 to 27, 20.5 to 26, 20.5 to 25, 20.5 to 24, 21 to 30, 21 to 29, 21 to 28, 21 to 27, 21 to 26, 21 to 25, 21 to 24, 21.5 to 30, 21.5 to 29, 21.5 to 28, 21.5 to 27, 21.5 to 26, 21.5 to 25, 21.5 to 24, 22 to 30, 22 to 29, 22 to 28, 22 to 27, 22 to 26, 22 to 25, 22 to 24, 22.5 to 30, 22.5 to 29, 22.5 to 28, 22.5 to 27, 22.5 to 26, 22.5 to 25, 22.5 to 24, 23 to 30, 23 to 29, 23 to 28, 23 to 27, 23 to 26, 23 to 25, 23 to 24, 23.5 to 30, 23.5 to 29, 23.5 to 28, 23.5 to 27, 23.5 to 26, 23.5 to 25, or 23.5 to 24.

In an optional embodiment, the thickness of the silica shell layer 12 may be 23.28 nm or 21.85 nm, and the thickness thereof may vary depending on whether or not ethanol pretreatment is performed.

Hereinafter, a composition for surface-enhanced Raman scattering spectroscopy will be described.

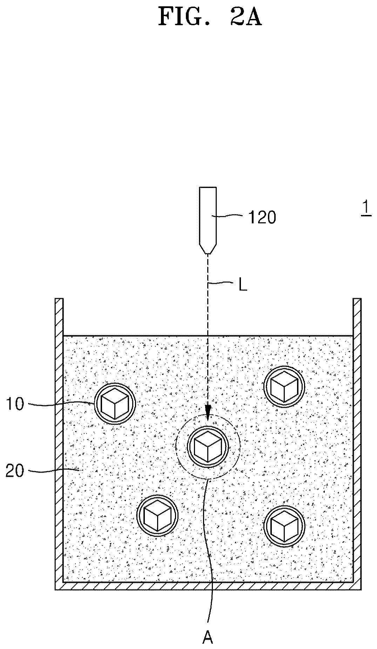

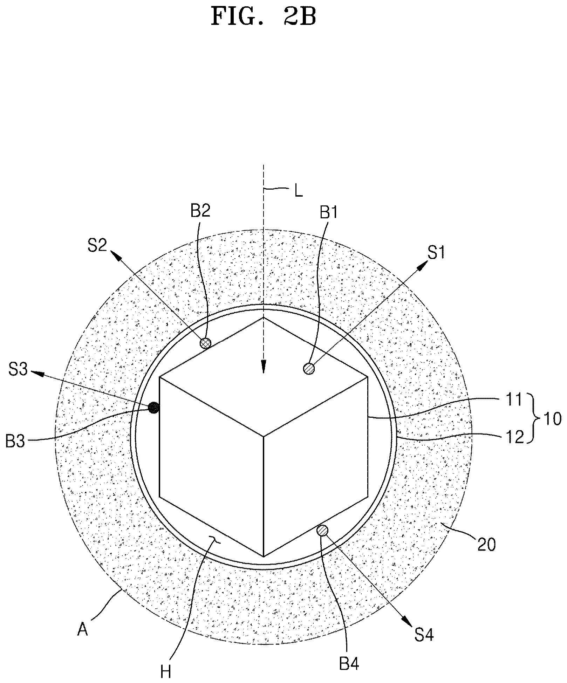

FIG. 2A illustrates a diagnostic solution used in a diagnostic method according to an embodiment, and FIG. 2B is an enlarged view of A region of FIG. 2A.

Referring to FIG. 2A, another embodiment of the present disclosure provides a composition for surface-enhanced Raman scattering spectroscopy (hereinafter, referred to as a "diagnostic solution"), including the dispersion 20 and the structures 10 for surface-enhanced Raman scattering spectroscopy (hereinafter, referred to as a "structure") dispersed in the dispersion 20. The composition may be used as a diagnostic solution 1 for surface-enhanced Raman scattering spectroscopy.

In an embodiment, the structure 10 may be dispersed in the dispersion 20 of the diagnostic solution 1, and for more specific and detailed descriptions of the structures 10, refer to the descriptions in the previous embodiments.

In an embodiment, the dispersion 20 may refer to a liquid-type substance in which nanoscale particles can be dispersed. The dispersion 20 is not particularly limited, and it should be interpreted that any liquid-type substance capable of dispersing the nanoparticles 11 that can be easily selected by a person skilled in the art falls within the scope of the present disclosure. In some embodiments, the dispersion 20 may be, for example, water (H₂O) or ethanol (EtOH), but the present disclosure is not limited to these examples.

In an embodiment, the diagnostic solution 1 may further include an additive. The additive may be, for example, a surfactant. The surfactant may allow nanoscale particles and/or the aforementioned structures 10 to be more uniformly dispersed in the dispersion 20.

In an embodiment, the surfactant is not particularly limited, a cationic surfactant and/or an anionic surfactant may be used, and it should be interpreted that any surfactant that can be easily selected by a person skilled in the art falls within the scope of the present disclosure. In some embodiments, the surfactant may be, for example, CTAB or CTAC, but the present disclosure is not limited to these examples.

Hereinafter, methods of preparing the structure 10 for surface-enhanced Raman scattering spectroscopy and the diagnostic solution 1 for surface-enhanced Raman scattering spectroscopy will be described.

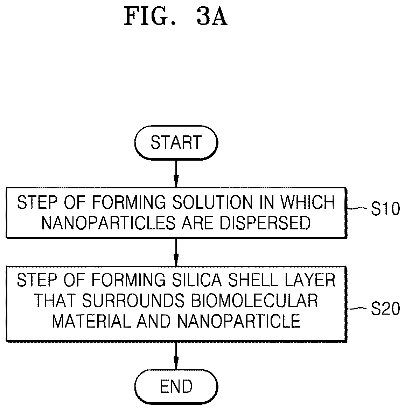

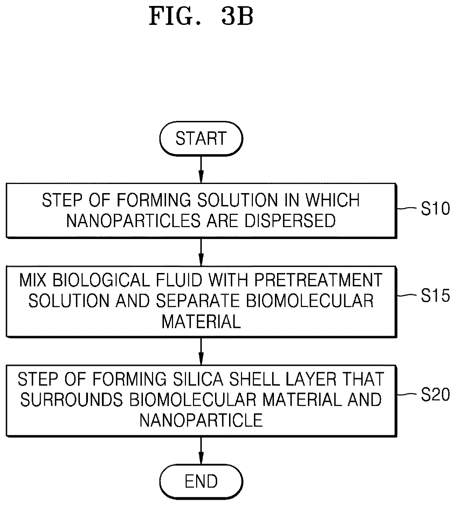

FIGS. 3A and 3B are flowcharts illustrating a method of preparing a structure for surface-enhanced Raman scattering spectroscopy, according to an embodiment.

Referring to FIG. 3A, to solve the technical problem, an embodiment of the present disclosure provides a method of preparing a structure for surface-enhanced Raman scattering spectroscopy (hereinafter, referred to as a "structure preparation method), the method including the steps of: forming a solution in which the nanoparticles 11 are dispersed (S10); and forming the silica shell layer 12 that surrounds each of the nanoparticles 11 (S20).

In an optional embodiment, the structure preparation method may include growing the nanoparticles 11 in a solution and forming the silica shell layer 12 that surrounds each of the nanoparticles 11, and therefore, a method of preparing the diagnostic solution 1 for surface-enhanced Raman scattering spectroscopy may also be provided.

In an embodiment, the formation of the solution (S10) may include a step of forming a mixed solution by mixing a first solution including a first precursor compound including a first plasmonic metal, and a second solution including a second precursor compound including a second plasmonic metal.

By mixing the first precursor compound and the second precursor compound, the first plasmonic metal of the first precursor compound may grow into the core 111, and the second plasmonic metal of the second precursor compound may grow into the second plasmonic metal shell layer 112 along an outer surface of the core 111. Accordingly, the core-shell nanoparticle 11 may be formed.

For more specific and detailed descriptions of the first plasmonic metal and the second plasmonic metal, refer to the descriptions in the previous embodiments.

In some embodiments, the formation of the core-shell nanoparticle 11 is not particularly limited, and it should be interpreted that any method that can be easily selected by a person skilled in the art falls within the scope of the present disclosure.

In an embodiment, the first precursor compound may include a first plasmonic metal, and may refer to a compound capable of forming nanoscale particles including a first plasmonic metal. The first precursor compound is not particularly limited, and it should be interpreted that any nanoscale precursor compound that can be easily selected by a person skilled in the art falls within the scope of the present disclosure. The first precursor compound may be, for example, HAuCl₄.3H₂O, but the present disclosure is not limited to the example.

In an embodiment, the second precursor compound may include a second plasmonic metal, and may refer to a compound capable of forming nanoscale particles or a shell of nanoparticles that include a second plasmonic metal. The second precursor compound is not particularly limited, and it should be interpreted that any precursor compound of nanoparticles that can be easily selected by a person skilled in the art falls within the scope of the present disclosure. The second precursor compound may be, for example, AgNO₃, but the present disclosure is not limited to the example.

In an embodiment, in case that the first solution and the second solution are mixed, any one or more of a reducing agent and a surfactant may be mixed together. In case that a mixed solution is formed, the first precursor compound may form the core 111 of the nanoparticle 11 through nanoscale growth, and in case that any one or more of a reducing agent and a surfactant are mixed together, the growth of the nanoparticle 11 may be further activated.

The reducing agent and the surfactant are not particularly limited, it should be interpreted that any reducing agent and any surfactant that can be easily selected by a person skilled in the art fall within the scope of the present disclosure, and examples of the reducing agent and the surfactant include NaBH₄ and CTAB and CTAC, respectively.

In an embodiment, the structure preparation method may further include a step of mixing the mixed solution with a coating solution including a water-soluble polymeric compound. In case that a water-soluble polymeric compound is mixed with the mixed solution including the nanoparticles 11, water-soluble polymeric compounds may form the polymer coating layer 113 on at least a region of the surface of each of the nanoparticles 11, and aggregation of the nanoparticles 11 may be prevented.

In an embodiment, examples of water-soluble polymeric compounds include, but are not limited to, polyvinylpyrrolidone, polyvinyl alcohol, polyfluorosulfonate, hydroxyethyl cellulose, hydroxypropyl cellulose, cellulose acetate, and polyamide.

The polymer coating layer 113 also enables a biomolecular material to be easily positioned on the surface of a metal, thereby providing superior sensitivity in case that a Raman signal is acquired. For example, blood contains various biomolecular materials such as nucleic acids, proteins, organic materials, and inorganic materials, and the polymer coating layer 113 may be formed on the surface of a nanoparticle and act as an intermediate material capable of forming molecular interaction between the nanoparticle and biomolecules so that these biomolecular materials can be positioned between the metallic nanoparticle and a silica shell layer.

A biomolecular material in blood may be attached to the intermediate material that is applied onto the surface of the metallic nanoparticle, through molecular interactions, and a silica shell layer starts to be formed on the surface of the metallic nanoparticle to which biomolecules are not attached and the biomolecules are immobilized without being detached in a process of surrounding the metallic nanoparticle, and as a result, various biomolecular materials in blood may exist in the accommodation space between the metallic nanoparticle and the silica shell layer.

In an optional embodiment, to form the polymer coating layer 113 on the surface of the nanoparticle 11, a mixed solution may be formed and after a predetermined period of time, may be mixed with a coating solution.

In an embodiment, the structure preparation method may further include a step of mixing the mixed solution with a coating solution including a polymeric compound including a unit represented by Formula 1, and in an optional embodiment, may further include a step of mixing the mixed solution with a coating solution including a water-soluble polymeric compound including a unit represented by Formula 1:

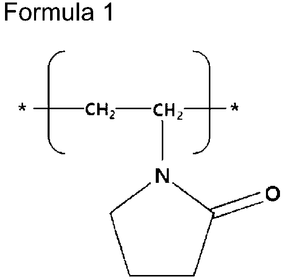

In an embodiment, the structure preparation method may include a step of mixing a solution or mixed solution in which the nanoparticles 11 are dispersed, with a third solution including the biomolecular material B and a fourth solution including a silica precursor compound. By mixing the solution including the nanoparticles 11 with the biomolecular material B and the silica precursor compound and causing a reaction to occur therebetween, the nanoparticle 11 and the biomolecular materials B may be arranged together inside the silica shell layer 12.

In an embodiment, the biomolecular material B and the silica precursor compound may be mixed in the solution including the nanoparticles 11 by using a stirrer and a reaction may be allowed to occur therebetween. As an optional example, the mixing may be performed using an orbital shaker or a seesaw shaker, thereby causing a reaction to occur therebetween.

For more specific and detailed descriptions of the biomolecular material B, refer to the descriptions in the previous embodiments.

In an embodiment, the silica precursor compound may refer to compounds capable of forming the silica shell layer 12 after reaction, and is not particularly limited, and it should be interpreted that any silica precursor compound that can be easily selected by a person skilled in the art falls within the scope of the present disclosure. The silica precursor compound may be, for example, TEOS, but the present disclosure is not limited to the example.

Referring to FIG. 3B, in an embodiment, the third solution including the biomolecular material B may be a solution that is treated with a pretreatment solution (S15). The pretreatment solution may include material having low polarity, and as an optional example, may include materials having lower polarity than water.

The third solution may be prepared by treating and/or processing a solution directly extracted from a living organism (hereinafter, referred to as a "biological fluid"). The biological fluid may be, for example, plasma, tissue fluid, urine, or the like.

These biological fluids may use water (H₂O) as a solvent, and in case that a biological fluid is mixed with a pretreatment solution including materials with low polarity, biomolecular materials B with different molecular weights in the biomolecular fluid may be separated according to the molecular weights thereof.

In an embodiment, the material having lower polarity than water may be, for example, an aprotic material, methanol, ethanol, acetone, or DMSO, but the present disclosure is not limited to these examples, and it should be interpreted that any material with lower polarity than water that can be easily selected by a person skilled in the art falls within the scope of the present disclosure.

In an embodiment, the third solution may include two or more types of biomolecular materials (B).

By separating and extracting the upper or lower region of a biological fluid treated with a pretreatment solution, the dimensions of the overall acquired surface-enhanced Raman scattering spectroscopic signals may be diversified, and thus, it is possible to organize a more detailed database for the biomolecular materials B. Ultimately, the structure 10 capable of more accurately diagnosing diseases may be provided.

The above-described embodiments of the present disclosure may also be applied in a manner combined with each other.

Another aspect of the present disclosure relates to a diagnostic method and diagnostic system using surface-enhanced Raman scattering spectroscopy.

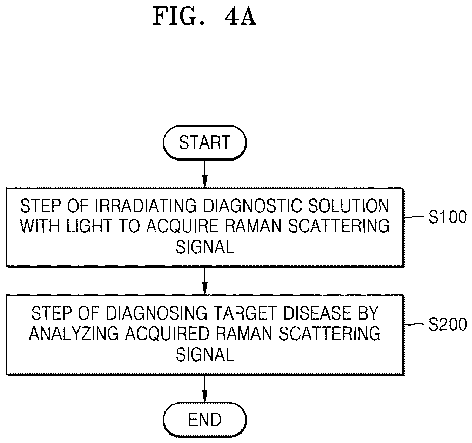

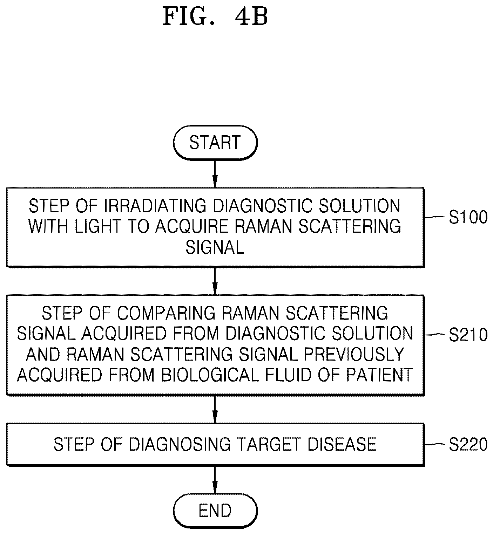

FIGS. 4A and 4B are flowcharts illustrating a diagnostic method using surface-enhanced Raman scattering spectroscopy, according to an embodiment.

Referring to FIG. 4A, to solve the technical problem, an embodiment of the present disclosure may provide a diagnostic method using surface-enhanced Raman scattering spectroscopy (hereinafter, referred to as "the diagnostic method), including: an irradiation step of irradiating the diagnostic solution 1 including the biomolecular material B with light to acquire a surface-enhanced Raman scattering spectroscopic signal (S100); and a step of analyzing the acquired surface-enhanced Raman scattering spectroscopic signal to diagnose a target disease (S200).

Biomolecular materials that appear specifically according to a specific disease may be present in a living organism with the specific disease. As such, a diagnostic method of diagnosing a target disease by using a specific surface-enhanced Raman scattering spectroscopic signal (hereinafter, referred to as a "spectroscopic signal") that can be acquired from biomolecular materials may be provided.

As described below, the diagnostic method according to an embodiment may include a step (not shown) of acquiring a first spectroscopic signal from a biological fluid extracted from a patient suffering from a disease or a solution including the same. In other embodiments, by acquiring first spectroscopic signals from patients suffering from various disease, spectroscopic information about specific spectroscopic signals according to the types of diseases may be organized into a database in a data storage unit 140 and the like, which will be described below.

The target disease may be, for example, pancreatic cancer, Alzheimer's disease, rheumatism, corona, influenza, or Alzheimer's dementia, but the present disclosure is not limited to these examples.

In an embodiment, the biological fluid refers to a liquid present in the human body, and may be, for example, blood, plasma, tissue fluid, lymphatic fluid, or urine. The biological fluid is not limited to the above examples, and it should be interpreted that any liquid in the human body that can be easily extracted by a general public, those of ordinary skill in the art, a medical professional, or a medical assistant falls within the scope of the present disclosure.

The diagnostic method according to an embodiment may include an irradiation step (S100) of irradiating the diagnostic solution 1 including the biomolecular material B with light to acquire a spectroscopic signal. As described above, structures for surface-enhanced Raman scattering spectroscopy may be dispersed in the diagnostic solution, and a composition for surface-enhanced Raman scattering spectroscopy in which the structures 10 for surface-enhanced Raman scattering spectroscopy as described above (hereinafter, referred to as a "structures") are dispersed may be used. In other embodiments, these diagnostic solutions may be transported in a produced state, or may be prepared at a location where a disease is diagnosed. For more specific and detailed descriptions related to the diagnostic solution, refer to the descriptions in the previous embodiments related to the structure 10 and the composition in which the structures 10 are dispersed.

In an embodiment, a second spectroscopic signal may be acquired by irradiating the diagnostic solution 1 including the biomolecular material B with light. In this regard, as described below, by comparative analysis of the second spectroscopic signal and pieces of spectroscopic information stored in the data storage unit 140 and the like, it is possible to diagnose which disease a patient has by using the diagnostic solution. In an optional embodiment, in the comparative analysis process as described above, it is possible to identify what biomolecular substances B are dissolved or dispersed in the diagnostic solution.

As described above, the diagnostic method according to an embodiment of the present disclosure may include acquiring a spectroscopic signal by directly irradiating a liquid diagnostic solution with light, and does not require a separate drying process, thus drastically reducing the time required for diagnosis.

In other embodiments, because the diagnostic method according to an embodiment involves diagnosing a disease or the like by directly irradiating the diagnostic solution 1 in a liquid phase with light L, the problem of contamination in the experiment environment may not occur significantly compared to existing diagnostic methods. Furthermore, the structures 10, which will be described below, dispersed in a liquid may maintain a similar environment even when experiments are repeated, thereby increasing reproducibility.

In other embodiments, the diagnostic solution 1 used in the diagnostic method according to an embodiment may be stored and transported in a liquid state, and thus convenience of storage and transportation of the diagnostic solution 1 may also be secured. Also, the diagnostic solution 1 may be prepared in a liquid state, and thus mass production of the diagnostic solution 1 is also possible.

In an embodiment, light L irradiated to the diagnostic solution 1 may be light of a wavelength and intensity used for surface-enhanced Raman scattering or Raman scattering, and may be, for example, laser light. However, the light is not limited to the above example.

Referring to FIG. 2A, in an embodiment, the diagnostic solution 1 may include the dispersion 20 and the structures 10 dispersed in the dispersion 20. As in the above-described structure, in the present disclosure, a spectroscopic signal may be acquired by irradiating the structures 10 dispersed in a liquid state with light L without drying the diagnostic solution 1, and the reproducibility and accuracy of the signal may be increased.

In other embodiments, the diagnostic solution 1 may be prepared by mixing with a biological fluid extracted from a patient suffering from a disease, thereby allowing the biomolecular materials B to be included in the structure 10 included in the diagnostic solution 1.

Referring to FIG. 2B, the structure 10 may include the biomolecular material B in a region of the inside thereof, and when light is irradiated between the biomolecular material B arranged inside the structure 10 and a region of the structure 10, a surface plasmon phenomenon occurs, thereby generating a specific second spectroscopic signal S according to the type of the biomolecular material B in a region of the structure 10. By comparative analysis of the second spectroscopic signal S and the above-described pieces of spectroscopic information, a target disease may be diagnosed.

In other embodiments, referring back to FIG. 2B, different biomolecular materials B1 to B4 may be included in a region of the inside of the structure 10. As such, the different biomolecular materials B1 to B4 generate different spectroscopic signals S1 to S4. In other embodiments, the generated spectroscopic signals S1 to S4 are spectroscopic signals that are specifically generated from the biomolecular materials B1 to B4.

By comparative analysis of the spectroscopic signals and the above-described pieces of spectroscopic information, a target disease may be diagnosed. In an optional embodiment, the spectroscopic signals S1 to S4 may be analyzed to identify the biomolecular materials B1 to B4.

As in the structure of the structure 10 of FIGS. 1A to 1E, by including the silica shell layer 12, aggregation of the structures 10 may be prevented, and the structures 10 may be well dispersed in the dispersion 20. In other embodiments, by arranging the nanoparticle 11 including a plasmonic metal and the biomolecular material B together inside the silica shell layer 12, a surface-enhanced Raman scattering spectroscopic signal may be generated more efficiently between the nanoparticle 11 and the biomolecular materials B.

Referring back to FIGS. 1E and 2B, in an embodiment, the biomolecular material B may include two or more types of biomolecular materials B1 to B4. As such, in case that different types of biomolecular materials B are included, specific surface-enhanced Raman scattering spectroscopic signals S1 to S4 corresponding to the biomolecular materials B may be generated.

In an embodiment, although the drawings illustrate the second plasmonic metal shell layer 112 as having a cubic shape, the second plasmonic metal shell layer 112 may have a regular polyhedral structure in addition to a regular hexahedron, and a regular polyhedron may include, for example, at least one of a regular octahedron, a regular dodecahedron, and a regular icosahedron.

In an embodiment, the second plasmonic metal shell layer 112 may include a second plasmonic metal, and a width of a plasmon resonance peak on a surface of the second plasmonic metal may be narrower than a width of a plasmon resonance peak on a surface of the first plasmonic metal.

As in the above-described structure, by making the width of a plasmon resonance peak on a surface of the second plasmonic metal narrower, in case that the structures 10 are dispersed in the dispersion 20, superior sensitivity may be provided in case that surface-enhanced Raman scattering spectroscopic signals are acquired.

In an optional embodiment, the width of a plasmon resonance peak on a surface of the first plasmonic metal and the width of a plasmon resonance peak on a surface of the second plasmonic metal may be measured under the same conditions.

Referring to FIGS. 1A to 1E, in an embodiment, the nanoparticle 11 may include the core 111, the second plasmonic metal shell layer 112, and the polymer coating layer 113 that surrounds the second plasmonic metal shell layer 112. The polymer coating layer 113 may allow the surfaces of the nanoparticles 11 to be negatively charged and prevent aggregation of the nanoparticles 11.

Due to having the above-described structure, in a step before the formation of the silica shell layer 12 in the method of preparing the structure 10, aggregation of the nanoparticles 11 may be prevented, and furthermore, the silica shell layer 12 may be formed more easily. For more detailed descriptions of the structure 10, refer to the related descriptions in the previous embodiments.

In an embodiment, the structure 10 may be formed by mixing a solution in which the nanoparticles 11 are dispersed, a solution including the biomolecular material B, and a solution including a silica precursor compound, to cause a reaction to occur therebetween. For more detailed descriptions of the preparation method for the structure 10, refer to the related descriptions in the previous embodiments.

In an embodiment, the solution including the biomolecular material B may be, for example, a biological fluid extracted from a living organism or a solution in which the biomolecular material B is mixed in a solvent, but the present disclosure is not limited to the examples. In some embodiments, the solution including the biomolecular material B may include a pretreated solution, and a solution used in such a pretreatment process may be pretreated with a solution having lower polarity than water.

By performing such a pretreatment process, the solution including the biomolecular material B may have various chemical or physical environments, and the dimensions of the overall acquired surface-enhanced Raman scattering spectroscopic signals may be diversified, and thus, it is possible to organize a more detailed database for the biomolecular materials B. Ultimately, it is possible to provide the structure 10 capable of more accurately acquiring surface-enhanced Raman scattering spectroscopic signals.

In an embodiment, the material having lower polarity than water may be, for example, an aprotic material, methanol, ethanol, acetone, or DMSO, but the present disclosure is not limited to these examples, and it should be interpreted that any material with lower polarity than water that can be easily selected by a person skilled in the art falls within the scope of the present disclosure.

In an embodiment, the preparation method for the structure 10 is not particularly limited, and it should be interpreted that any preparation method for the structure 10, which can be easily selected by a person skilled in the art, falls within the scope of the present disclosure. For example, the structure 10 may be prepared by using the principle of growing metal particles into nanoparticles in a solution, and may be prepared by stirring a solution including the nanoparticles 11 having a core-shell structure, TEOS, and solutions including biomolecular materials. Preferably, the structure 10 may follow the manufacturing method described above for the structure for surface-enhanced Raman scattering spectroscopy and the diagnostic method.

Referring back to FIG. 4A, the diagnostic method may include a step (S200) of diagnosing a target disease by analyzing the second spectroscopic signal acquired through the diagnostic solution 1 in step S100. In an optional embodiment, the diagnostic method may include a step (S220) of diagnosing a target disease by comparative analysis of the second spectroscopic signal acquired from the diagnostic solution and spectroscopic information (S210) (see FIG. 4B).

As described above, biomolecular materials that appear specifically according to a specific disease may be distributed in the body of a patient suffering from the specific disease. A specific spectroscopic signal may be acquired depending on the biomolecular material B inside the structure 10 according to an embodiment, and the specific spectroscopic signal may be analyzed, thereby diagnosing a target disease. In an optional embodiment, by identifying the biomolecular material B, a target disease may be diagnosed.

The diagnostic method according to an embodiment may include a step (not shown) of acquiring a first spectroscopic signal from a biological fluid extracted from a patient suffering from a specific disease or a solution including the same. As an optional example, the first spectroscopic signal may be organized into a database and pieces of spectroscopic information may be stored in the data storage unit 140 and the like.

In this regard, a method of acquiring the first spectroscopic signal is not particularly limited, any surface-enhanced Raman scattering method using nanoparticles, a substrate, a liquid phase, a solid phase, or the like may be used, and it should be interpreted that any method of acquiring a surface-enhanced Raman scattering spectroscopic signal that can be easily selected by a person skilled in the art falls within the scope of the present disclosure.

In an optional embodiment, the diagnostic method may include a step of acquiring a spectroscopic signal for the biomolecular material B, organizing the spectroscopic signal for the biomolecular material B into a database DB, and storing spectroscopic information. In this regard, pieces of spectroscopic information organized into a database may be stored in the data storage unit 140, as in embodiments described below.

In another optional embodiment, the organization of the spectroscopic signal for the biomolecular material B into a database may include a step of organizing the biomolecular materials B that cause any disease into a database by acquiring spectroscopic signals by using bacteria and/or viruses that cause any disease.

The biomolecular materials B may exist specifically in a living organism depending on the type of disease, and it is possible to provide a method of diagnosing a target disease by analyzing the type of the biomolecular material B from various perspectives. Such biomolecular materials B may be not only proteins and sugars, but also bacteria, viruses, and the like, as described above.

In an embodiment, referring back to FIG. 2B, the biomolecular material B may include two or more different types of biomolecular materials B. Depending on the type of disease, biomolecules that specifically occur in a living organism may also include two or more types of biomolecular materials B. As such, by identifying the biomolecular materials B, the accuracy and reproducibility of diagnosis of a target disease may be further increased.

In an embodiment, a method of comparing and analyzing the first spectral signal and the second spectral signal may include a method of comparing and confirming the intensities of peaks, the degrees of matching, specific wave numbers, and the like.

For example, in comparing the first spectroscopic signal and the second spectroscopic signal, as for pancreatic cancer patients, in case that a surface-enhanced Raman scattering signal is acquired, a spectroscopic signal having peaks appearing at 1453 cm⁻¹, 878 cm⁻¹, 1517 cm⁻¹, 1385 cm⁻¹, 1303 cm⁻¹, 1153 cm⁻¹, 1184 cm⁻¹, 1043 cm⁻¹, 669 cm⁻¹, and 435 cm⁻¹ may be acquired, and in case that a spectroscopic signal appears at 1453 cm⁻¹, 878 cm⁻¹, 1517 cm⁻¹, 1385 cm⁻¹, 1303 cm⁻¹, 1153 cm⁻¹, 1184 cm⁻¹, 1043 cm⁻¹, 669 cm⁻¹, and 435 cm⁻¹ in a diagnostic solution as described above, it may be diagnosed that the biomolecular materials included in the diagnostic solution as described above are derived from patients with pancreatic cancer.

In an embodiment, the diagnostic method may further include a step of comparing with a comparative experimental group. It can be confirmed whether a spectroscopic signal acquired using the diagnostic solution 1 is obtained through normal operation of a diagnostic system 100, and the comparative experimental group may consist of the same solvent as the diagnostic solution 1.

In some embodiments, the step of comparing with a comparative experimental group is not particularly limited, and it should be interpreted that any method that can be easily selected by a person skilled in the art falls within the scope of the present disclosure.

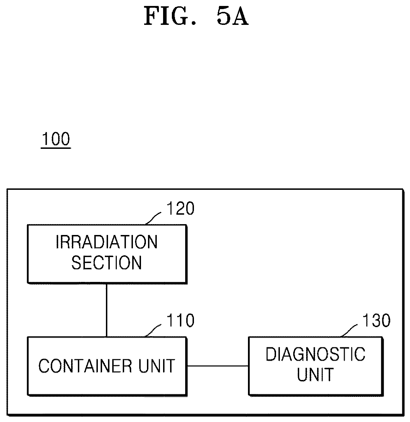

FIGS. 5A to 5C are block diagrams illustrating a diagnostic system 100 according to an embodiment.

To solve the technical problem, another embodiment of the present disclosure provides a disease diagnostic system 100 using surface-enhanced Raman scattering spectroscopy (hereinafter, referred to as "the diagnostic system").

Referring to FIG. 5A, the diagnostic system 100 according to an embodiment may include a container unit 110 in which the diagnostic solution 1 is placed, an irradiation section 120 that irradiates the diagnostic solution 1 placed in the container unit 110 with light to acquire a spectroscopic signal from a region of the structure 10, and a diagnostic unit 130 that analyzes the acquired spectroscopic signal.

In an embodiment, the diagnostic system 100 may further include a preparation unit that prepares the structure 10. The structure 10 including the biomolecular material B in a region of the inside thereof may be prepared by the preparation unit.

In an optional embodiment, the diagnostic solution 1 in which the structures 10 are dispersed may be prepared by the preparation unit. As such, the prepared diagnostic solution 1 may be used in the container unit 110, the irradiation section 120, and the like, which will be described below.

A method of preparing the structure 10 by the preparation unit is not particularly limited, and for more specific and detailed descriptions related to the preparation method for the structure 10, refer to the descriptions in the previous embodiments.

In an embodiment, the diagnostic solution 1 may include the structure 10. In the diagnostic system 100, the diagnostic solution 1 may be directly irradiated with light by the irradiation section 120 to acquire a spectroscopic signal from a region of the structure 10 arranged in the diagnostic solution 1, and the spectroscopic signal may be analyzed by the diagnostic unit 130 to diagnose a target disease.

In some embodiments, as described above, the structure 10 may include the biomolecular material B, and for more specific and detailed descriptions thereof, refer to the descriptions in the previous embodiments.

In an embodiment, the container unit 110 may be a configuration in which the diagnostic solution 1 is placed, and the diagnostic solution 1 placed in the container unit 110 may be irradiated with light to allow a spectroscopic signal to be acquired from a region of the structure 10. It is preferable that the diagnostic solution 1 is placed sufficiently, and as an optional example, the container unit 110 in which the diagnostic solution 1 is placed may have a volume of at least 200 µL, but the present disclosure is not limited to the volume.

The container unit 110 is not particularly limited and can be easily selected by a person skilled in the art, and it should be interpreted that any container allowing a liquid to be accommodated therein to generate a spectroscopic signal falls within the scope of the present disclosure.

In an embodiment, the irradiation section 120 is configured to irradiate the diagnostic solution 1 with light. In case that the diagnostic solution 1 is irradiated with light, a spectroscopic signal is generated from a region of the inside of the structure 10 arranged in the diagnostic solution 1.

In an optional embodiment, the structure 10 may include the nanoparticle 11, the biomolecular material B, and a silica shell that surrounds the nanoparticle 11 and the biomolecular material B. In other embodiments, a spectroscopic signal may be generated near a contact surface between the biomolecular material B and the nanoparticle 11. For more specific and detailed descriptions of the structure 10, refer to the descriptions in the previous embodiments.

Referring back to FIG. 5A, in an embodiment, the irradiation section 120 may include an irradiation unit that emits light. Irradiation units are not particularly limited, and in an optional embodiment, the irradiation unit may include a laser irradiation unit.

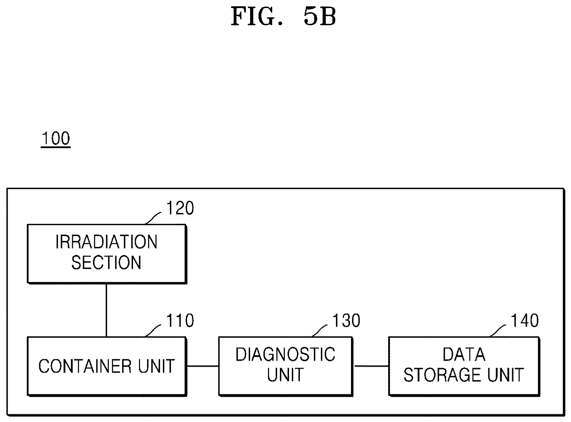

Referring to FIG. 5B, in an embodiment, the diagnostic unit 130 may diagnose a target disease by comparative analysis of spectroscopic information in the foregoing embodiments and second spectroscopic signals. In an optional embodiment, the biomolecular material B included in the diagnostic solution 1 may be identified during this process.

In some embodiments, in case that the diagnostic unit 130 uses information about spectroscopic signals, spectroscopic information about the first spectroscopic signal stored in the data storage unit 140, which will be described below, may be used.

In an embodiment, the diagnostic unit 130 may use an algorithm that compares and/or analyzes the degree of wavenumber matching of peaks, the intensities of the peaks, the degree of matching and the like between spectroscopic information and the second spectroscopic signal, the first spectroscopic signal may be searched for through the process, and a target disease that specifically corresponds to the found first spectroscopic signal may be diagnosed.

Referring to FIG. 3C, in an embodiment, the diagnostic unit 130 may include a first diagnostic unit 130a that searches for the first spectroscopic signal corresponding to the second spectroscopic signal from among spectroscopic information, and a second diagnostic unit 130b that diagnoses a disease by using the found first spectroscopic signal.

Referring back to FIG. 5B, in an embodiment, the diagnostic system 100 may include the data storage unit 140 that acquires spectroscopic signals and stores spectroscopic information.

In an embodiment, the data storage unit 140 may store pieces of spectroscopic information about the above-described first spectroscopic signals. The diagnostic unit 130 may search for the first spectral signal corresponding to the second spectral signal from among the pieces of spectroscopic information by comparing the second spectroscopic signal with the pieces of spectroscopic information stored in the data storage unit 140. In other embodiments, a specific disease may be searched for through the first spectroscopic signal, and thus a target disease may be diagnosed by the diagnostic unit 130.

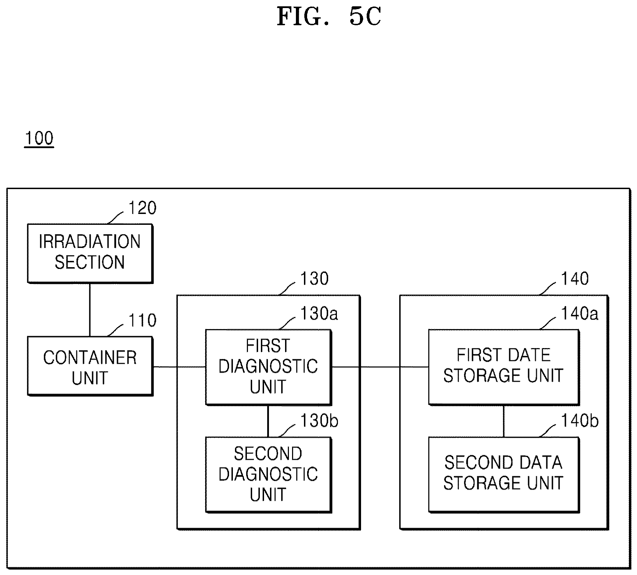

Referring back to FIGS. 5B and 5C, in an embodiment, the data storage unit 140 may include a first data storage unit 140a that stores spectroscopic information about the first spectroscopic signal. The spectroscopic information may include, for example, a spectrum chart of surface-enhanced Raman scattering spectroscopic signals for a specific disease, spectroscopic intensity, and set values of the irradiation section, but the present disclosure is not limited to these examples.

In an embodiment, the data storage unit 140 may include a second data storage unit 140b that stores information about diseases and the first spectroscopic signal. The diagnostic unit 130 may diagnose a target disease by using information recorded in the second data storage unit 140b.

In an optional embodiment, the data storage unit 140 may organize pieces of information about the biomolecular material B into a database. Information about the spectroscopic signal of the biomolecular material B may be, for example, a specific Raman scattering spectroscopic signal that occurs depending on the type of protein, bacteria, virus, mineral, or the like. For descriptions of a method of obtaining such information, refer to the descriptions in the previous embodiments.

In an embodiment, the data storage unit 140 and the diagnostic unit 130 may communicate with each other by using a wired/wireless communication method, and by using such a method, it is possible to provide the diagnostic system 100 for diagnosing a disease through near/far-distance methods. Such wired/wireless communication methods are not particularly limited, and it should be interpreted that any communication method that can be easily selected by a person skilled in the art falls within the scope of the present disclosure.

The above-described embodiments of the present disclosure may also be applied in a manner combined with each other.

Hereinafter, preparation examples, examples and experimental examples of the present disclosure will be described. However, these preparation examples, examples and experimental examples are intended to more specifically describe the configurations and effects of the present disclosure and are not intended to limit the scope of the present disclosure.

### Examples

### Example 1 - Structure including nanoparticle having core-shell structure

In Example 1, a structure for surface-enhanced Raman scattering spectroscopy and a composition for surface-enhanced Raman scattering spectroscopy in which the structures are dispersed were prepared. The specific preparation processes are as follows.

To prepare the structure for surface-enhanced Raman scattering spectroscopy and the composition for surface-enhanced Raman scattering spectroscopy in which the structures are dispersed, a nanoparticle was formed, and then a biomolecular material positioned near the formed nanoparticle and a silica shell layer that surrounds the nanoparticle and the biomolecular material together were formed.

### (1) Formation of core-shell nanoparticles

To form 2-3 nm-sized gold nanoparticles capped with CTAB, 9.75 ml of a 100 mM CTAB solution was prepared, and then 250 µl of a 10 mM HAuCl₄·3H₂O solution and 600 µl of a 10 mM NaBH₄ solution were added and stirred for 2 minutes, and then a reaction was allowed to occur therebetween for 3 hours.

Subsequently, to grow the formed 2-3 nm-sized gold nanoparticles capped with CTAB into 30 nm of gold nanoparticles, 16 mL of a 200 mM CTAC solution was prepared, and then 16 mL of a 0.5 mM HAuCl₄·3H₂O solution and 16 mL of a 0.8 mM ascorbic acid solution were added to 40 µL of the prepared CTAB-capped AuNP seeds, and then a reaction was allowed to occur therebetween for 7 days.

Thereafter, to form core-shell nanoparticles by forming second plasmonic metal shell layers of silver on the surfaces of the prepared gold nanoparticles with a size of 30 nm, 10 mL of a 1.0 mM AgNO₃ solution and 10 mL of a 25 mM ascorbic acid solution were added to the formed 30 nm AuNP solution, and then a reaction was allowed to occur therebetween for 40 minutes. Next, after centrifugation, the resulting solution was redispersed in triple distilled water, thereby completing the formation of Au-Ag core-shell nanoparticles.

### (2) PVP coating onto surfaces of nanoparticles having core-shell structure

10 mL of a solution of the formed Au-Ag core-shell nanoparticles was prepared, and then 0.5 mL of a 1 v/v% SDS solution and 5 mL of a 2.5 %(w/v) PVP solution were added, and a reaction was allowed to occur therebetween for 10 minutes, followed by centrifugation and redispersion in distilled water, thereby forming core-shell nanoparticles having PVP coating layers formed on the surfaces thereof.

### (3) Formation of biomolecular material and silica shell layer

To form the previously formed core-shell nanoparticle having a PVP coating layer, a biomolecular material positioned near the nanoparticle, and a silica shell layer that surrounds both the nanoparticle and the biomolecular material, the process was carried out in the following order. In Example 1.1, plasma was pretreated with ethanol, and then a structure for surface-enhanced Raman scattering spectroscopy was prepared. In Example 1.2, a structure for surface-enhanced Raman scattering spectroscopy was prepared using plasma without the pretreatment process.

### Example 1.1 - With pretreatment

To perform plasma pretreatment, 10 µL of plasma was prepared, and then 20 µL of deionized water and 70 µL of ethanol (EtOH) were added, and a reaction was allowed to occur therebetween for 10 minutes.

17.5 µL of the core-shell nanoparticles having PVP coating layers, 20 µL of an ethanol-pretreated plasma supernatant, 44.5 µL of deionized water, and 20 µL of a 0.05 v/v% TEOS solution were added, and a reaction was allowed to occur therebetween for 30 minutes, followed by addition of 20 µL of a 10 v/v% DMA solution, thereby completing the preparation of a composition for surface-enhanced Raman scattering spectroscopy in which the structures for surface-enhanced Raman scattering spectroscopy are dispersed.

### Example 1.2 - Without pretreatment

17.5 µL of the core-shell nanoparticles having PVP coating layers, 2 µL of plasma without pretreatment with ethanol, 60.5 µL of deionized water, and 20 µL of a 0.05 v/v% TEOS solution were added, and a reaction was allowed to occur therebetween for 30 minutes, followed by addition of 20 µL of a 10 v/v% DMA solution, thereby completing the preparation of a composition for surface-enhanced Raman scattering spectroscopy in which the structures for surface-enhanced Raman scattering spectroscopy are dispersed.

### Experimental Example 1 - Confirmation of structure

In Experimental Example 1, experiments were conducted to confirm the conditions of the structures for surface-enhanced Raman scattering spectroscopy prepared according to Examples 1. 1 and 1.2.

### Experimental Example 1.1 - Confirmation of size of nanoparticles

In Experimental Example 1.1, an experiment was conducted to confirm the size of nanoparticles. The specific experimental methods are measurement of the size of nanoparticles using dynamic light scattering and TEM imaging.

As a result of measurement of the size of nanoparticles using dynamic light scattering, a spherical gold nanocore had a diameter of 31 nm and a PI of 0.16. The diameter of cubic Au-Ag core-shell nanoparticles was 47.5 nm and the PI thereof was 0.25.

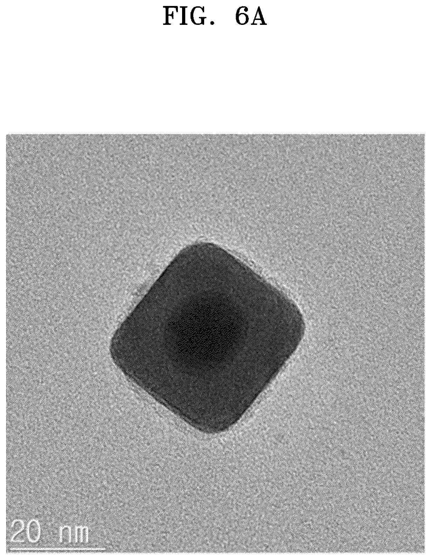

Referring to an TEM image of FIG. 6A, the width of the Au-Ag core-shell nanoparticle was measured to be approximately 30 nm, and it was confirmed that a spherical gold core was present inside a cubic silver metal shell layer.

### Experimental Example 1.2 - Confirmation of silica shell layer

In Experimental Example 1.2, an experiment was conducted to confirm whether a silica shell layer was formed on a surface of each structure. The specific experimental methods are measurement of the size of nanoparticles using dynamic light scattering and TEM imaging.

Upon ethanol pretreatment, the diameter of a nanoparticle with a silica shell layer, as measured by dynamic light scattering, is 90.25 nm and the PI thereof is 0.35, and in case that ethanol pretreatment is not performed, the diameter and PI thereof are 87.4 nm and 0.31. The thickness of a silica shell is 23.28 nm upon ethanol pretreatment, and is 21.85 nm without ethanol pretreatment.

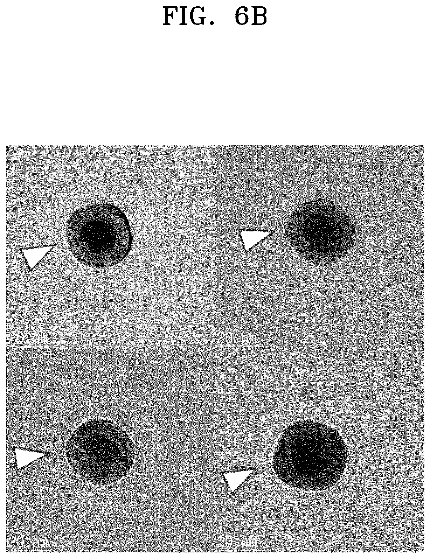

Referring to TEM images of FIG. 6B, it was confirmed that a silica shell layer was formed outside an Au-Ag core-shell structure consisting of a gold core positioned at the center thereof and a cubic silver metal shell layer that surrounds the gold core.

### Experimental Example 1.3 - Confirmation of biomolecular material

In Experimental Example 1.3, Raman scattering spectra in the presence or absence of a biomaterial in the structures were compared.

As a specific experimental method, the difference between the Raman spectra of a case (red) where a plasma sample was added to PVP-coated silver (Ag) nanocube particles and a silica shell was formed and a case (blue) where a silica shell was formed without adding a plasma sample was confirmed.

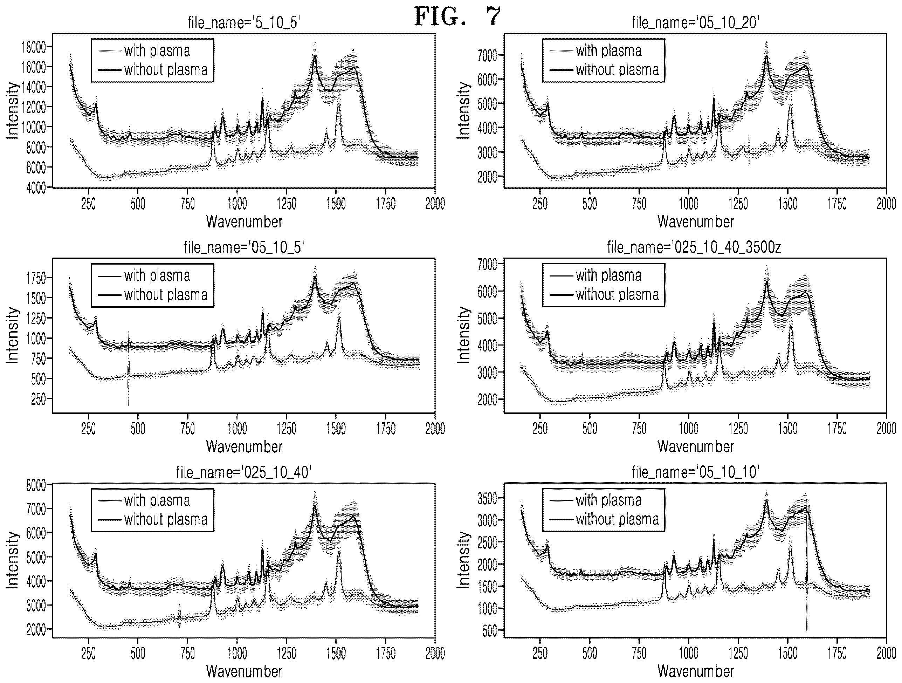

FIG. 7 illustrates the experimental results of Experimental Example 1.3.

Referring to FIG. 7, as a result of confirming the difference between Raman spectra in cases where a plasma sample, i.e., a biomaterial, is present or absent in nanostructures, it can be confirmed that a plurality of peaks newly appear or disappear in the spectra. For example, peaks at 1170 cm⁻¹ and 1520 cm⁻¹ newly appear due to the presence of plasma, and peaks at 930cm⁻¹ and 1130cm⁻¹ disappear.

### Experimental Example 2 - Experiment to confirm Raman spectra with or without pretreatment

In Experimental Example 2, an experiment was conducted to confirm what differences occur in the Raman spectra depending on whether ethanol pretreatment was performed or not, through Examples 1.1 and 1.2.

As a specific experimental method, a process of forming a silica shell together with plasma and nanoparticles was performed, and after mixing 70% ethanol and plasma at a 9:1 (v/v) ratio and leaving it for 10 minutes, a process of forming a silica shell together with the nanoparticles was performed, and Raman spectra were obtained for both cases and compared.

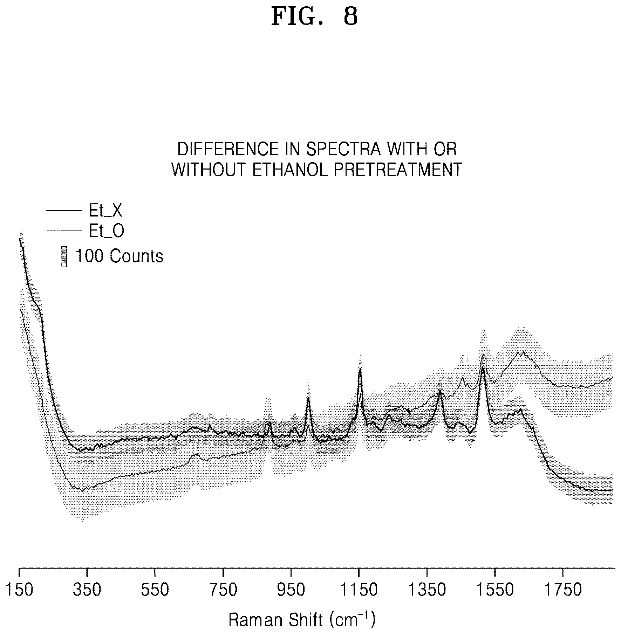

FIG. 8 illustrates Raman spectra showing the experimental results of Experimental Example 2.

Referring to FIG. 8, as a result of comparing the case without pretreatment (blue) and the case with ethanol pretreatment (orange), it can be seen that peaks appear at 650cm⁻¹ and 880cm⁻¹ upon pretreatment, whereas peaks appear at 1280 cm⁻¹ and 1390 cm⁻¹ in the case where pretreatment was not performed.

### Experimental Example 3 - Confirmation of surface-enhanced Raman scattering spectroscopic signal for diagnosis of target disease

In Experimental Example 3, a surface-enhanced Raman scattering spectroscopic signal was acquired by using the diagnostic solution prepared according to Example 1.

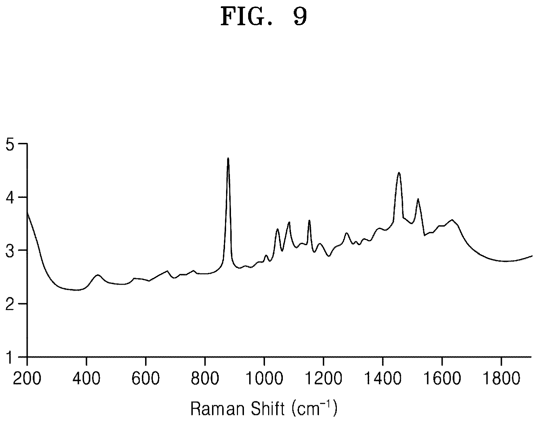

The diagnostic solution was placed in a 96-well plate, irradiated with a 532 nm laser, and the scattered light was acquired using a Raman spectrometer. The results of confirmation are illustrated in FIG. 9.

### Experimental Example 4 - Confirmation of diagnostic error rate of target disease

In Experimental Example 4, a target disease was diagnosed by using the spectroscopic signal acquired through Experimental Example 3. As a result of confirming the peaks appearing at 1453 cm⁻¹, 878 cm⁻¹, 1517 cm⁻¹, 1385 cm⁻¹, 1303 cm⁻¹, 1153 cm⁻¹, 1184 cm⁻¹, 1043 cm⁻¹, 669 cm⁻¹, and 435 cm⁻¹, normal individuals and cancer patients were classified with an accuracy of 0.931, a sensitivity of 0.800, and a specificity of 0.800.

The foregoing description of the present disclosure is provided only for illustrative purposes, and it will be understood by one of ordinary skill in the art to which the present disclosure pertains that the disclosure may be embodied in various modified forms without departing from the technical spirit or essential characteristics thereof. Thus, the embodiments described herein should be considered in an illustrative sense only and not for the purpose of limitation. For example, each component described to be of a single type may be implemented in a distributed manner. Likewise, components described as distributed may be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A structure for surface-enhanced Raman scattering spectroscopy, comprising:
a silica shell layer having an accommodation space therein; and
a nanoparticle comprising a plasmonic metal,
wherein the nanoparticle is arranged in a region of the accommodation space in the silica shell layer.

2. The structure of claim 1, wherein
the nanoparticle comprises a core including a first plasmonic metal, and a second plasmonic metal shell layer disposed to surround the core and including a second plasmonic metal,
wherein the second plasmonic metal shell layer has a symmetric structure with respect to a center of the second plasmonic metal shell layer.

3. The structure of claim 1, wherein
the structure comprises a biomolecular material arranged in the accommodation space.

4. The structure of claim 2, wherein
the first plasmonic metal and the second plasmonic metal each independently comprise any one or more selected from the group consisting of Au, Ag, Cu, Al, W, Pt, Ni, and Pd.

5. The structure of claim 2, wherein
a width of a plasmon resonance peak on a surface of the second plasmonic metal is narrower than a width of a plasmon resonance peak on a surface of the first plasmonic metal.

6. The structure of claim 2, wherein
the second plasmonic metal shell layer has a regular polyhedral structure.

7. A method of preparing a structure for surface-enhanced Raman scattering spectroscopy, the method comprising:
preparing a solution in which nanoparticles are dispersed; and
forming a silica shell layer that surrounds each of the nanoparticles dispersed in the solution.

8. The method of claim 7, wherein
the preparation of the solution in which nanoparticles are dispersed comprises forming a mixed solution by mixing a first solution and a second solution, the first solution including a first precursor compound including a first plasmonic metal, and the second solution including a second precursor compound including a second plasmonic metal, and the nanoparticle comprises a core including a first plasmonic metal, and a second plasmonic metal shell layer disposed to surround the core and including a second plasmonic metal,
wherein the second plasmonic metal shell layer has a symmetric structure with respect to a center of the second plasmonic metal shell layer.

9. The method of claim 8, further comprising
mixing the mixed solution with a coating solution including a water-soluble polymeric compound.

10. The method of claim 8, wherein
the formation of the silica shell layer comprises mixing the mixed solution with a third solution and a fourth solution, the third solution including a biomolecular material and the fourth solution including a silica precursor compound, and
the silica shell layer is formed such that the nanoparticle and at least a part of the biomolecular material are arranged together inside the silica shell layer.

11. A disease diagnostic method using surface-enhanced Raman scattering spectroscopy, comprising:
acquiring a first surface-enhanced Raman scattering spectroscopic signal from a biological fluid extracted from a patient;
irradiating a diagnostic solution, in which the structure for surface-enhanced Raman scattering spectroscopy according to any one of claims 1 to 6 is dispersed, with light to acquire a second surface-enhanced Raman scattering spectroscopic signal from a region of the structure for surface-enhanced Raman scattering spectroscopy; and
identifying a disease of the patient by comparing the first surface-enhanced Raman scattering spectroscopic signal and the second surface-enhanced Raman scattering spectroscopic signal.

12. The disease diagnostic method of claim 11, wherein
the structure for surface-enhanced Raman scattering spectroscopy comprises two or more types of biomolecular materials.

13. The disease diagnostic method of claim 11, further comprising
organizing, into a database, the first surface-enhanced Raman scattering spectroscopic signal acquired from the biological fluid extracted from the patient.

14. The disease diagnostic method of claim 11, further comprising
acquiring a surface-enhanced Raman scattering spectroscopic signal for a biomolecular material in the structure for surface-enhanced Raman scattering spectroscopy, and organizing, into a database, information about the surface-enhanced Raman scattering spectroscopic signal for the biomolecular material.

15. A disease diagnostic system using surface-enhanced Raman scattering
spectroscopy, comprising:
a container unit in which a diagnostic solution is placed;
an irradiation section that irradiates the diagnostic solution placed in the container unit with light, and generates a surface-enhanced Raman scattering spectroscopic signal from a region of the structure for surface-enhanced Raman scattering spectroscopy according to any one of claims 1 to 6 included in the diagnostic solution; and
a diagnostic unit that analyzes the generated surface-enhanced Raman scattering spectroscopic signal.

16. The disease diagnostic system of claim 15, wherein
the structure for surface-enhanced Raman scattering spectroscopy comprises two or more types of biomolecular materials.

17. The disease diagnostic system of claim 15, further comprising
a data storage unit that acquires the surface-enhanced Raman scattering spectroscopic signal and stores spectroscopic information about the surface-enhanced Raman scattering spectroscopic signal.
